# EUROPÄISCHE PATENTANMELDUNG

(11) **EP 1 659 107 A1**
(43) Veröffentlichungstag der Anmeldung: **24.05.2006**
(21) Anmeldenummer: 05024255.1
(22) Anmeldetag: 08.11.2005
(51) Int. Cl.: C07C 45/50, C07C 47/02

(54) **Verfahren zur Herstellung von Aldehyden**

(30) Priorität: 19.11.2004 DE 102004055832
(71) Anmelder: Celanese Chemicals Europe GmbH, 61476 Kronberg (DE)
(72) Erfinder: Thiel, Dietmar, 46147 Oberhausen (DE); Frohning, Carl Dieter, 46485 Wesel (DE)
(74) Vertreter: Szameitat, Jürgen

(57) **Zusammenfassung**

Verfahren zur Hydroformylierung von Olefinen durch Verknüpfung der bei der Hydroformylierungsreaktion herrschenden Eingangsgrößen mit den Zielgrößen einer Hydroformylierungsreaktion, wobei die Verknüpfung mittels mindestens eines künstlichen neutronalen Netzes erfolgt.

## Beschreibung

Die vorliegende Erfindung betrifft ein Verfahren zur Hydroformylierung von Olefinen unter Verwendung eines künstlichen neuronalen Netzes zur Kontrolle und zur Steuerung.

Als Hydroformylierung bezeichnet man die übergangsmetallkatalysierte Umsetzung von Olefinen mit Wasserstoff und Kohlenmonoxid zu Aldehyden und Alkoholen, die ein Kohlenstoff-Atom mehr als das eingesetzte Olefin enthalten. Die Hydroformylierung besitzt erhebliche wirtschaftliche und technische Bedeutung, weltweit werden gegenwärtig mehr als 6 Mio. t/a an Produkten durch Hydroformylierungsverfahren hergestellt. Die dabei primär erhaltenen Aldehyde werden als solche verwendet oder stellen wertvolle Vorprodukte für die Gewinnung von beispielsweise Alkoholen, Carbonsäuren, Estern oder Aminen dar.

Die Hydroformylierung wird durch Hydridometallcarbonyle, vorzugsweise solcher der Metalle der VIII. Nebengruppe des Periodensystems, katalysiert. Neben Kobalt, dem klassischen Katalysatormetall, werden seit einigen Jahren zunehmend Katalysatoren auf Basis von Rhodium eingesetzt. Im Gegensatz zu Kobalt gestattet es Rhodium, die Reaktion bei niedrigerem Druck durchzuführen. Darüber hinaus werden bei Einsatz endständiger Olefine bevorzugt geradkettige n-Aldehyde und nur in untergeordnetem Maße iso-Aldehyde gebildet. Schließlich ist auch die Hydrierung der eingesetzten Olefine zu gesättigten Kohlenwasserstoffen in Gegenwart von Rhodium-Katalysatoren deutlich niedriger als bei Anwendung von Kobalt-Katalysatoren.

Industriell realisiert ist die Hydroformylierung olefinisch ungesättigter Verbindungen unter der katalytischen Wirkung von Rhodiumcarbonyl-Komplexen mit tertiären organischen Phosphinen oder Phosphiten als Liganden. Bei einer Prozessvariante arbeitet man in homogener organischer Phase, d.h., eingesetztes Olefin, Katalysator und Reaktionsprodukte liegen gemeinsam in Lösung vor. Die Reaktionsprodukte werden aus dem Gemisch meist destillativ, seltener nach anderen Verfahren wie der Extraktion, abgetrennt. Das in homogener Phase durchgeführte Hydroformylierungsverfahren kann in Form eines Gaskreislaufverfahrens gemäß US 4,247,486 oder in Form eines Flüssigkeitskreislaufverfahrens gemäß US 4,148,830 ausgestaltet werden.

Eine weitere Verfahrensvariante ist durch die Gegenwart einer wässrigen Katalysatorphase gekennzeichnet, die Rhodiumcarbonyl-Komplexe und wasserlösliche organische Phosphine enthält. Diese Ausführungsform ist z.B. aus DE-B-26 27 354 bekannt. Ihr besonderer Vorteil ist die leichte Trennung von organischem Reaktionsprodukt und wässriger Katalysatorphase, z.B. durch Phasentrennung. Diese Trennung erfolgt schonend und ohne Anwendung thermischer Verfahrensschritte und Verluste lassen sich vermeiden, die durch Folgereaktionen der entstandenen Aldehyde eintreten können. Weiterhin erzielt man sehr hohe Ausbeuten und bei der Verwendung unverzweigter endständiger Olefine erhält man ganz überwiegend unverzweigte Aldehyde. Aufgrund des Vorliegends einer flüssigen organischen Phase und einer wässrigen Katalysatorphase bezeichnet man diese Hydroformylierungsvariante auch als heterogenes oder zweiphasiges Verfahren. Eine bewährte Ausführungsform dieser Arbeitsweise ist beispielsweise in der EP-B1-0 103 810 beschrieben.

Sowohl das in homogener Phase durchgeführte Hydroformylierungsverfahren als auch das heterogene Hydroformylierungsverfahren, das auch als Ruhrchemie/Rhone-Poulenc-Verfahren bekannt ist, haben sich in der großtechnischen Anwendung etabliert und werden in der Literatur, beispielsweise von C.D.Frohning, C.W.Kohlpaintner in B.Cornils/W.A.Herrmann, Applied Homogeneous Catalysis with Organometallic Compounds, Volume 1, 1. Auflage, Seiten 29-104, VCH Weinheim, 1996, ausführlich behandelt.

Die nach den erwähnten Varianten technisch betriebenen Verfahren sind über viele Jahre hinweg laufend verbessert worden und weisen daher einen hohen Reifegrad auf, was sich in guter Stoff- und Energie-Nutzung sowie hoher Betriebssicherheit zu erkennen gibt. So werden für die Hydroformylierung von Propylen Umsatzgrade im Bereich oberhalb 85%, bezogen auf eingesetztes Propylen, genannt, wobei zugleich ein Verhältnis von n-Butanal zu iso-Butanal von über 90/10 erzielt wird.

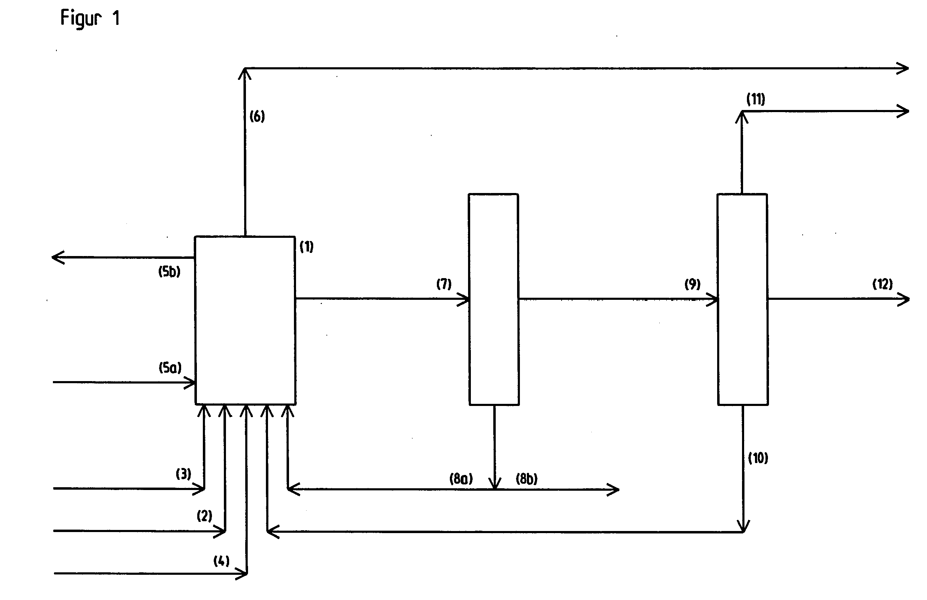

Figur 1 zeigt ein Prinzipschema eines Hydroformylierungsverfahrens. In den gekühlten und gerührten Reaktor (1) werden Olefin (2) und Synthesegas (3), ein Gemisch von Wasserstoff und Kohlenmonoxid, eingespeist. Eine weitere Zuführung (4) dient zur Ergänzung des Katalysator-Instandes, wenn dies erforderlich ist. Die bei der Hydroformylierung freigesetzte Wärme wird über ein geeignetes Kühlmedium (5a und 5b) abgeführt. Um die Ansammlung von Inerten zu begrenzen, wird aus dem Reaktor (1) ein Abgasstrom (6) entnommen. Nicht umgesetzte Einsatzprodukte, Katalysator und Reaktionsprodukte werden aus dem Reaktor (1) abgeführt und in einer ersten Folgestufe (7) aufgetrennt, aus der der ausgetragene Katalysator (8a) abgenommen und in den Reaktor (1) zurückgeführt wird. Bei Bedarf wird ein Teil des Katalysators (8b) ausgeschleust und zur Aufarbeitung und/oder Reaktivierung gegeben. Aus dem nach Katalysatorabtrennung erhaltenen Reaktionsgemisch werden in einer weiteren Folgestufe (9) nicht umgesetztes Olefin und Synthesegas sowie durch Hydrierung des Olefins gebildete Alkane abgetrennt und dem Reaktor als Rückgas (10) wieder zugeführt, wobei ein Teilstrom (11) als Abgas abgeführt werden muß, um den Aufbau von Inerten im Reaktionssystem zu begrenzen. Die Reaktionsprodukte (12) werden der Weiterverarbeitung zugeführt.

Schon aus diesem stark vereinfachten Schema ist ersichtlich, dass Steuerung und Kontrolle eines solchen Hydroformylierungsverfahrens keine triviale Aufgabe darstellen. Allein die Erfassung und Regelung der großen Zahl der Mengenströme und der damit zusammenhängenden Verweilzeiten sowie die Einstellung und die Regelung von Temperatur, Druck und Konzentrationen an einer Vielzahl von Stellen im Verfahren bedeuten bereits erhebliche Anforderungen. Es kommt erschwerend hinzu, daß ein Hydroformylierungskatalysator keine über die Betriebszeit konstante Aktivität aufweist, sondern in seiner Leistungsfähigkeit durch unvermeidbare Alterungsvorgänge verändert wird. Auf diese Änderungen muß die Regelung reagieren, zum Beispiel durch Änderung der jeweiligen Betriebstemperatur, der Einsatzprodukt-Mengen oder der Verweilzeiten, um die Produktionsleistung der Anlage konstant zu halten. Auch die kontinuierliche oder schrittweise Anhebung des Rhodium-Instandes oder der Liganden-Konzentration gehört zu den Aufgaben der Verfahrenslenkung, wie z.B. aus B.Cornils, E.Wiebus, Chem.-Ing.-Techn. 1994, 66,196; E.Wiebus, B.Cornils, Chemtech 1995, 25,33 bekannt.

Aus B. Cornils in J.Falbe (Ed.), New Syntheses with Carbon Monoxide, Springer-Verlag, Berlin 1980, 1. Auflage, ist dem Fachmann bekannt, daß die Reaktionsparameter für die kontinuierliche Durchführung einer Hydroformylierungsreaktion nicht frei wählbar sind. Als Beispiele seien folgende allgemein bekannte Abhängigkeiten angeführt:
(a) Ein hoher Wasserstoff-Partialdruck wirkt sich positiv auf die Reaktionsgeschwindigkeit aus. Zugleich nimmt jedoch die Hydrierung des Olefins zum Alkan zu.
(b) Ein hoher Kohlenmonoxid-Partialdruck wirkt sich nachteilig auf die Reaktionsgeschwindigkeit und auf das normal/iso-Verhältnis der gebildeten Aldehyde aus und beschleunigt den Liganden-Abbau, stabilisiert jedoch Rhodiumcarbonylhydride.
(c) Eine hohe Reaktionstemperatur wirkt sich positiv auf die Reaktionsgeschwindigkeit, jedoch negativ auf die Stabilität des Katalysators aus.
(d) Ein hoher Überschuss an Ligand wirkt sich günstig auf das normal/iso-Verhältnis der gebildeten Aldehyde aus, ist jedoch nachteilig für die Reaktionsgeschwindigkeit.

Schon diese wenigen Beispiele verdeutlichen, dass eine große Zahl von Parametern für eine sichere, technisch und wirtschaftlich erfolgreiche Durchführung der Hydroformylierungsreaktion zu erfassen und zu regeln ist, wobei diese Parameter zum Teil voneinander abhängig sind und/oder sich gegenseitig beeinflussen. Bei der technischen Durchführung des Hydroformylierungsprozesses von Olefinen besteht somit das Erfordernis, eine Vielzahl von Verfahrensparametern so aufeinander abzustimmen und zu steuern, dass die gewünschten Werte für angestrebte Zielgrößen, bespielsweise für die Aldehydausbringung oder für das Verhältnis von normal- zu iso-Aldehyd sicher eingestellt werden können.

Obgleich die Hydroformylierung bereits seit vielen Jahrzehnten im technischen Maßstab durchgeführt wird, muß es überraschen, daß bisher kein allgemein anwendbares mathematisches Modell bekannt geworden ist, mit dem die Reaktion zwischen Olefin, Kohlenmonoxid und Wasserstoff

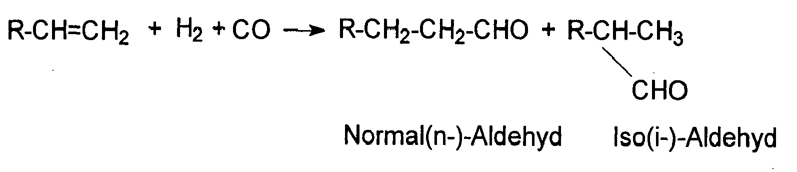

in Abhängigkeit von den Verfahrensparametern wie Temperaturen, Partialdrücken von Olefin, Wasserstoff und Kohlenmonoxid, Konzentration an Katalysator und Ligand, nach Geschwindigkeit und Ausmaß quantitativ beschrieben werden kann. Zwar sind in der Literatur Beispiele für Reaktionsgeschwindigkeits-Gleichungen angegeben, beispielsweise nach S.S.Divekar, R,M.Deshpande, R.V.Chaudari, Catal.Lett. **1993**,21,191, jedoch wurden diese unter idealisierten Bedingungen erstellt und genügen nicht den Anforderungen an eine sichere technische Reaktionsführung.

Besondere Schwierigkeiten bereitet die Modellierung des heterogenen Hydroformylierungsverfahrens in Gegenwart einer wässrigen Katalysatorlösung, bei dem neben der chemischen Reaktion auch die Stoffübergänge zwischen der Gasphase, der flüssigen organischen Phase und der ebenfalls vorliegenden flüssigen wässrigen Katalysatorphase und dementsprechend auch der Einfluß der Durchmischung aller drei Phasen zu berücksichtigen ist (K.-D. Wiese et al., Catalysis Today 79-80 (2003) 97-103).

Somit erfolgte bisher die Kontrolle und Steuerung eines Verfahrens zur Hydroformylierung von Olefinen mittels der durch lange Betriebszeiten erworbenen empirischen Daten.

Es sind Ansätze bekannt geworden, diesen auf empirische Daten basierenden und letztlich nicht völlig zufriedenstellenden Zustand zu verbessern.

So behandelt EP-B1- 589 463 ein Verfahren zur Kontrolle des normal/iso-Verhältnisses der entstehenden Aldehyde in einer Hydroformylierungs-Reaktion zwischen einem Olefin, Wasserstoff und Kohlenmonoxid (CO) durch Steuerung des CO-Partialdruckes im Reaktionssystem. Nach der beanspruchten Arbeitsweise wird in einem Reaktionssystem der Zielwert für den CO-Partialdruck dadurch eingehalten, dass entweder die Fließgeschwindigkeit des dem System zugeführten Synthesegases (Gemisch aus CO und Wasserstoff) oder die Fließgeschwindigkeit des das System verlassenden Abgases geregelt wird. Das bekannte Verfahren beschreibt zunächst die Erfassung des CO-Partialdruckes, dann die Erzeugung von Steuergrößen, die aus der Differenz zwischen Soll- und Ist-Wert des CO-Partialdruckes resultieren, und schließlich die Nutzung dieser Steuergrößen zur Beeinflussung der Fließgeschwindigkeit, wobei wiederum entweder die Fließgeschwindigkeit des dem System zugeführten Synthesegases oder die Fließgeschwindigkeit des das System verlassenden Abgases beeinflußt wird. Nach dem bekannten Verfahren wird nur eine Eingangsgröße, nämlich der CO-Partialdruck, mit einer Ausgangsgröße, nämlich mit dem normal/iso-Verhältnis der entstehenden Aldehyde, verknüpft, wobei die Höhe des einzuhaltenden CO-Partialdruckes vom Anwender willkürlich vorgegeben wird. Weitere Verfahrensparameter und Zielgrößen werden nicht berücksichtigt.

Bei SU 1 527 234 erfolgt die Regelung der Einsatzmengen von Synthesegas und Propylen in der Propylenhydroformylierung mit dem Ziel, die benötigte Menge an Synthesegas niedrig zu halten. Es wird eine regelungstechnische Einrichtung beschrieben, in der die jeweiligen Volumina von Einsatzgas und Abgas miteinander verknüpft werden. Weitere Verfahrensparameter und Zielgrößen werden nicht berücksichtigt.

Aufgabe der vorliegenden Erfindung ist es demnach, ein Hydroformylierungsverfahren bereitzustellen, bei dem die zu berücksichtigenden Verfahrensparameter, auch Eingangsgrößen genannt, mit den Zielgrößen des Hydroformylierungsverfahrens, auch Ausgangsgrößen genannt, quantitativ verknüpft werden, so dass die vorgegebenen Werte für die Zielgrößen durch die Festlegung der zugehörigen Verfahrensparameter erreicht werden. Dadurch kann das Hydroformylierungsverfahren kontrolliert und gesteuert werden.

Diese Aufgabe wird gelöst durch ein Verfahren zur Hydroformylierung von Olefinen durch Verknüpfung der bei der Hydroformylierungsreaktion herrschenden Eingangsgrößen mit den Zielgrößen einer Hydroformylierungsreaktion, dadurch gekennzeichnet, dass die Verknüpfung mittels mindestens eines künstlichen neuronalen Netzes erfolgt.

Während die konventionellen Ansätze für eine verbesserte Kontrolle und Steuerung der Hydroformylierungsreaktion darauf beruhen, eine einzige maßgebliche Größe, oder jedenfalls möglichst wenige solcher maßgeblichen Größen, zu erfassen und zu regeln, ermöglicht der erfindungsgemäße Einsatz mindestens eines künstlichen neuronalen Netzes, im folgenden auch mit KNN abgekürzt, eine gänzlich andere, umfassendere Vorgehensweise.

Der Aufbau und die Funktion eines KNN gestatten es, eine große Zahl von Verfahrensparametern als Eingangsgrößen zu verarbeiten und daraus ein Modell zu erstellen, das den gewichteten Einfluß jedes Parameters auf Verlauf und Ergebnis des Verfahrens quantitativ beschreibt. Als Eingangsgrößen können Daten herangezogen werden, die im vorangegangenen Verlauf des Hydroformylierungsverfahrens bereits gewonnen worden waren. Bei dieser Vorgehensweise wird das KNN trainiert und/oder nachtrainiert.

Vorzugsweise wird jedoch das KNN in der Weise eingesetzt, dass Daten aus dem laufenden Hydroformylierungsprozeß in das KNN eingespeist werden, wobei das KNN mit der Zeit fortlaufend nachtrainiert wird und selbständig die Qualität des intern erzeugten Modells an die Gegebenheiten des laufenden Hydroformylierungsverfahren anpaßt und verbessert. Man macht sich somit die Fähigkeit von KNN zum selbstständigen Lernen während des laufenden Hydroformylierungsverfahrens zu nutze.

Ein weiterer Vorteil dieser Vorgehensweise besteht darin, dass keinerlei Eingriffe in das Hydroformylierungsgeschehen selbst notwendig sind, sondern dass das KNN lediglich auf solche Daten zugreift, die bisher bereits zur Kontrolle und Steuerung des Hydroformylierungsverfahrens erfaßt werden.

Ein wesentlicher Bestandteil der vorliegenden Erfindung besteht darin, dass das KNN die Eingangsgrößen gewichtet, wobei Größe und Vorzeichen der Gewichte der Bedeutung der Eingangsgröße für den Wert der Zielgrößen entsprechen.

Eine Besonderheit des KNN besteht ferner darin, dass die für die Verknüpfung der Eingangsgröße mit den Zielgrößen erforderlichen Algorithmen nicht bekannt sein müssen, sondern dass das KNN die Gewichte aus den eingegebenen Daten selbst ableiten kann. Ein KNN kann somit trainiert, nachtrainierten oder fortlaufend nachtrainierten werden, entweder auf Basis bereits vorhandener Daten oder aber auf Basis solcher Daten, die während des laufenden Prozesses erfasst und in das KNN eingespeist werden. Ein KNN besitzt demnach die Fähigkeit, komplexe Zusammenhänge zwischen den Eingangsgrößen oder den Verfahrensparametern und den Ausgangsgrößen oder Zielgrößen des Hydroformylierungsverfahrens selbständig in einen Algorithmus umzuwandeln, der diese Zusammenhänge quantitativ beschreibt. Der einmal gewonnene Algorithmus ermöglicht dann Vorhersagen für die Zielgrößen aus neuen Datensätzen für die Eingangsgrößen.

Bei der Durchführung von Hydroformylierungsreaktionen sind dem Fachmann die prinzipiellen Abhängigkeiten zwischen den Eingangsgrößen oder Reaktionsparametern und den Zielgrößen wie Olefin-Umsatz, normal/iso-Verhältnis der gebildeten Aldehyde, Bildung von Alkanen und Hochsiedern oder Katalysator-Desaktivierung zwar grundsätzlich bekannt. Aber erst mit der erfinderischen Verwendung eines KNN können die Abhängigkeiten zwischen den einzelnen Prozessparametern und damit ihre gegenseitige Beeinflussung erfaßt und quantifiziert werden. Damit können erstmals die komplexen Zusammenhänge des Hydroformylierungsgeschehens in ihrer Gesamtheit erfasst und durch ein mathematisches Modell quantitativ beschrieben werden.

KNN sind kommerziell erhältlich und können mit einer frei verfügbaren Anzahl von Eingängen verknüpft werden, so dass eine entsprechend große Zahl von Eingangsgrößen ausgewählt werden kann. Diese Eingangsgrößen sind im Prinzip frei wählbar, jedoch wird man zweckmäßigerweise nur solche Eingangsgrößen auswählen, deren Bedeutung für das Ergebnis des Verfahrens, also für die Zielgrößen, bekannt ist oder deren Bedeutung gezielt untersucht werden soll.

Für ein Hydroformylierungs-Verfahren gemäß Figur 1 können beispielsweise folgende Eingangsgrößen und Zielgrößen oder Ausgänge festgelegt werden.

Beispiele für Eingangsgrößen oder Verfahrensparameter sind: Einsatzmenge an Olefin (2), Einsatzmenge an Synthesegas (3), Temperatur im Reaktor (1) Konzentration des Katalysators im Reaktor (1), Konzentration an Liganden im Reaktor (1), Partialdruck Olefin im Reaktor (1), Partialdruck Wasserstoff im Reaktor (1), Partialdruck Kohlenmonoxid im Reaktor (1), Volumen Abgas (6) abgeführt aus dem Reaktor (1), Menge Rohprodukt (7) abgeführt aus dem Reaktor (1), Volumen Katalysator-Kreislauf (8), Temperatur des Katalysator-Kreislaufs (8), Volumen Abgas (11) abgeführt aus der Gasabtrennung (9), Volumen Rückgas (10) zurückgeführt in den Reaktor (1) aus der Gasabtrennung (9).

Beispiele für Zielgrößen oder Ausgänge sind: Masse Rohaldehyde, normal/iso-Verhältnis der Aldehyde, Masse gebildetes Alkan, Masse gebildeter Hochsieder, Olefinumsatz.

Die erforderlichen Daten können jeder geeigneten Quelle entnommen werden, beispielsweise aus vorhandenen Datensätzen. Vorzugsweise jedoch werden die Eingangsgrößen während des laufenden Hydroformylierungsverfahrens einem Prozeßleitsystem oder einem sonstigen Prozeßdaten-Erfassungssystem entnommen und in geeigneter Weise dem KNN zugeführt, beispielsweise durch eine on-line Anbindung an eine Datenquelle. Eine zwischenzeitliche Speicherung der Daten auf einem Datenbankserver kann bei großen Datenmengen Vorteile bieten.

Das KNN wird zu einem als geeignet angesehenen Zeitpunkt mit ausgewählten Daten versehen und so lange trainiert, bis das erzeugte Modell die Zusammenhänge zwischen Eingangsgrößen und Zielgrößen mit der geforderten oder angestrebten Genauigkeit quantitativ beschreibt.

Der über das KNN ermittelte Algorithmus dient anschließend als Basis zur Durchführung einer Optimierungsrechnung mit einem konventionellen Modell, wobei die Verfahrensparameter für dieses konventionelle Modell auf den Bereich beschränkt werden, der durch die bei der Hydroformylierungsreaktion herrschenden Eingangsgrößen für das KNN bestimmt wird. Häufig enthalten kommerziell erhältliche KNN bereits eine integrierte Funktion zur Durchführung von Optimierungsrechnungen mit dem vom KNN erzeugten Algorithmus. Ebenfalls ist es auch möglich, den vom KNN erzeugten Algorithmus an ein übliches, kommerziell erhältliches Modell, beispielsweise an ein Tabellenkalkulationsprogramm, zu übergeben und in diesem Programm die Optimierungsrechnungen durchzuführen. Ebenso können die auf Basis des vom KNN erzeugten Algorithmus erhaltenen Daten per Hand durch den Anlagenfahrer für die Optimierung der Verfahrensparameter verarbeitet werden.

Die so optimierten Verfahrensparameter werden anschließend im technischen Hydroformylierungsbetrieb eingestellt.

### Beispiel 1

Beispiel 1 demonstriert die Anwendung eines KNN auf Daten aus dem Verfahren zur Propylenhydroformylierung mit dem Ziel, einen Algorithmus zu gewinnen, der die Zielgröße in Abhängigkeit von mehreren Verfahrensparametern oder Eingangsgrößen quantitativ beschreibt.

Es wurde eine kontinuierliche Hydroformylierung von Propylen nach dem heterogenen Zweiphasenverfahren durchgeführt. Als Ligand für den in Wasser gelösten Rhodium-Katalysator diente Tris-(phenyl-m-sulfosäure-Natrium)-Phosphin (TPPTS). Die Konzentration an Rhodium in der wässrigen Katalysatorlösung betrug 255 ppm, das Molverhältnis Ligand/Rhodium betrug 87/1. Über einen Zeitraum von 15 Tagen wurden durchschnittlich folgende Ergebnisse erhalten:

| | | |
|---|---|---|
| (1) | Temperatur Reaktor [°C] | 129,2 |
| (2) | Temperatur Katalysator-Kreislauf [°C] | 129,9 |
| (3) | Temperatur Gemisch Frischgas+Kreisgas Eintritt Reaktor [°C] | 99,7 |
| (4) | Einsatz Synthesegas (H₂ + CO) [Nm³ pro m³ Katalysator und Stunde] | 96 |
| (5) | Einsatz Propylen [kg pro m³ Katalysator und Stunde] | 101,7 |
| (6) | Partialdruck Propylen im Reaktor [MPa] | 2,0 |
| (7) | Ausbringen Rohaldehyde [kg pro m³ Katalysator und Stunde] | 144,3 |

[unter (3) bedeutet Frischgas das dem Hydroformylierungsreaktor zugeführte Gemisch aus Synthesegas und Propylen während Kreisgas das aus dem Prozeß abgetrennte und in den Reaktor zurückgeführte Gemisch aus Synthesegas, Propylen und Alkanen bedeutet, Bezugszeichen (10) gemäß Abbildung 1); unter (4) bedeutet Nm³ Normkubikmeter; bei Normtemperatur Tₙ = 273,15 K und Normdruck Pₙ = 101 325 Pa]

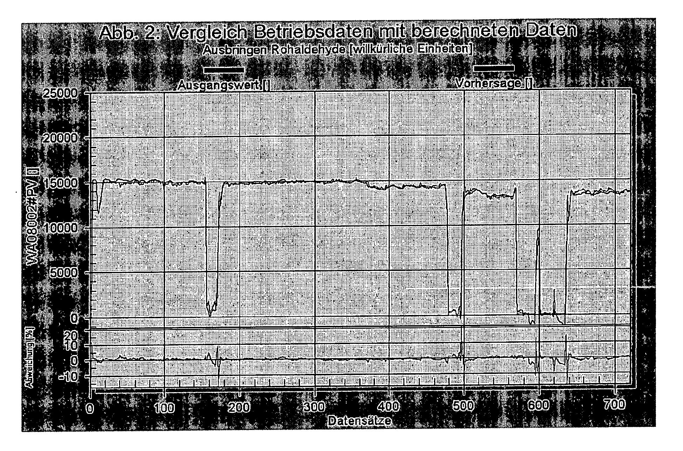

Alle 30 Minuten wurde für die Positionen (1) bis (4) sowie für (6) und (7) ein vollständiger Datensatz erstellt, insgesamt wurden so 722 Datensätze gesammelt. Betriebsbedingte Unterbrechungen oder Störungen wurden in diese Datensätze einbezogen, so dass ein typischer Ablauf festgehalten wurde. Die 722 Datensätze wurden in ein KNN (Neuromodel 2.0; Fa. Atlan-tec, D 47877 Willich-Münchheide) eingegeben, wobei die Positionen (1) bis (4) sowie (6) als Eingangsgrößen, Position (7) als Ausgangs- oder Zielgröße definiert wurden. Das Training des KNN mit den 722 Datensätzen führte zu einem Algorithmus, der es ermöglichte, die Zielgröße aus den Eingangsgrößen mit einem mittleren Fehler von 1,1 % vom Wertebereich der Zielgröße zu berechnen. Figur 2 verdeutlicht die Übereinstimmung zwischen gemessenen und berechneten Werten für die Zielgröße, besonders auch unter Einbeziehung betriebsbedingter Unterbrechungen des Verfahrens.

### Beispiel 2

Beispiel 2 zeigt die positive Auswirkung der gemäß Vorausberechnung abgestimmten Verfahrensparameter auf die Zielgröße.

Mit dem gemäß Beispiel 1 gewonnenen Algorithmus wurde eine Optimierungsrechnung für die ausgebrachte Menge an Rohaldehyden mit kommerziell erhältlicher Software durchgeführt. Dazu diente die in dem kommerziell erhältlichen KNN der Firma Neuromodel 2.0 der Fa. Atlan-tec, D-47877 Willich-Münchheide, integrierte Funktion "Genetischer Optimierer® ", wobei die Verfahrensparameter auf den durch die Eingangsgrößen bestimmten Bereich beschränkt waren. Der Rhodiumgehalt in der wässrigen Katalysatorlösung und das Molverhältnis TPPTS-Ligand/Rhodium wurden nicht verändert. Es wurden folgende Ergebnisse erhalten:

| | | Berechnet | Bestwert |
|---|---|---|---|
| (1) | Temperatur Reaktor [°C] | 133,2 | 133,0 |
| (2) | Temperatur Katalysator-Kreislauf [°C] | 130,4 | 125,4 |
| (3) | Temperatur Gemisch Frischgas+Kreisgas Eintritt Reaktor [°C] | 97,1 | 110,2 |
| (4) | Einsatz Synthesegas (H₂ + CO) [Nm³ pro m³ Katalysator und Stunde] | 134 | 108 |
| (5) | Einsatz Propylen [kg pro m³ Katalysator und Stunde] | 126,8 | 109,9 |
| (6) | Partialdruck Propylen im Reaktor [MPa] | 2,03 | 2,02 |
| (7) | Ausbringen Rohaldehyde [kg pro m³ Katalysator und Stunde] | 206,9 | 172,2 |

Der Bestwert bezeichnet die höchste Ausbringung an Rohaldehyden, die in dem betrachteten Betriebszeitraum gemäß Beispiel 1 unter Einsatz des KNN erhalten worden war. Die Einstellung des vorausberechneten Betriebszustandes ergab folgendes Ergebnis:

| | | Berechnet | Erhalten |
|---|---|---|---|
| (1) | Temperatur Reaktor [°C] | 133,2 | 133,0 |
| (2) | Temperatur Katalysator-Kreislauf [°C] | 130,4 | 129,4 |
| (3) | Temperatur Gemisch Frischgas+Kreisgas Eintritt Reaktor [°C] | 97,1 | 98,3 |
| (4) | Einsatz Synthesegas (H₂ + CO) [Nm³ pro m³ Katalysator und Stunde] | 134 | 129 |
| | | 134 | 129 |
| (5) | Einsatz Propylen [kg pro m³ Katalysator und Stunde] | 126,8 | 122 |
| (6) | Partialdruck Propylen im Reaktor [MPa] | 2,0 | 2,0 |
| (7) | Ausbringen Rohaldehyde [kg pro m³ Katalysator und Stunde] | 206,9 | 196 |

Der Vergleich der über den vom KNN erstellten Algorithmus berechneten Werte mit den im Hydroformylierungsverfahren erhaltenen Werten verdeutlicht, daß bei sorgfältiger Abstimmung mehrerer vorausberechneter Verfahrensparameter (Eingangsgrößen) aufeinander die Zielgröße "Ausbringung Rohaldehyde" nachhaltig beeinflußt werden kann. So führt eine Anpassung der Reaktionsparameter (1)-(4) und (6) zu einer deutlichen Erhöhung der Ausbringung an Rohaldehyden von 144,3 kg pro m³ Katalysator und Stunde (Beispiel 1) auf 196 kg pro m³ Katalysator und Stunde (Beispiel 2).

## Patentansprüche

1. Verfahren zur Hydroformlierung von Olefinen durch Verknüpfung der bei der Hydroformylierungsreaktion herrschenden Eingangsgrößen mit den Zielgrößen einer Hydroformylierungsreaktion, **dadurch gekennzeichnet, dass** die Verknüpfung mittels mindestens eines künstlichen neuronalen Netzes erfolgt.

2. Verfahren gemäß Anspruch 1, **dadurch gekennzeichnet, dass** das künstliche neuronale Netz nach der Hydroformylierungsreaktion aus den bei der Hydroformylierungsreaktion herrschenden Eingangsgrößen trainiert und/oder nachtrainiert wird.

3. Verfahren gemäß Anspruch 1, **dadurch gekennzeichnet, dass** das künstliche neuronale Netz während der Hydroformylierungsreaktion aus den bei der Hydroformylierungsreaktion herrschenden Eingangsgrößen fortlaufend nachtrainiert wird.

4. Verfahren gemäß Anspruch 3, **dadurch gekennzeichnet, dass** das fortlaufende Nachtrainieren des neuronalen Netzes mit den bei der Hydroformylierungsreaktion herrschenden Eingangsgrößen erfolgt, die aus einem Prozessdaten-Erfassungssystem entnommen werden.

5. Verfahren gemäß einem oder mehreren der Ansprüche 1 bis 4, **dadurch gekennzeichnet, dass** das künstliche neuronale Netz die Eingangsgrößen entsprechend ihrer Bedeutung für den Wert der Zielgrößen gewichtet.

6. Verfahren gemäß einem oder mehreren der Ansprüche 1 bis 5, **dadurch gekennzeichnet, dass** die mittels eines künstlichen neuronalen Netzes ermittelte Verknüpfung zwischen Eingangsgrößen und Zielgrößen mit einem konventionellen Modell kombiniert wird, das eine Optimierung der Zielgröße auf Basis des durch die Eingangsgrößen bestimmten Bereiches vornimmt.

7. Verfahren gemäß einem oder mehreren der Ansprüche 1 bis 6, **dadurch gekennzeichnet, dass** ein solches künstliches neuronalen Netz verwendet wird, dessen Zielgröße die Aldehydausbringung ist.

8. Verfahren gemäß einem oder mehreren der Ansprüche 1 bis 6, **dadurch gekennzeichnet, dass** ein solches künstliches neuronalen Netz verwendet wird, dessen Zielgröße das Verhältnis von normal zu iso-Aldehyd ist.

9. Verfahren gemäß einem oder mehreren der Ansprüche 1 bis 6, **dadurch gekennzeichnet, dass** ein solches künstliches neuronalen Netz verwendet wird, dessen Zielgröße die Menge an gebildeten Alkanen ist.

10. Verfahren gemäß einem oder mehreren der Ansprüche 1 bis 6, **dadurch gekennzeichnet, dass** ein solches künstliches neuronalen Netz verwendet wird, dessen Zielgröße die Menge an gebildeten Hochsiedern ist.

11. Verfahren gemäß einem oder mehreren der Ansprüche 1 bis 6, **dadurch gekennzeichnet, dass** ein solches künstliches neuronalen Netz verwendet wird, dessen Zielgröße der Olefinumsatz ist.

12. Verfahren gemäß einem oder mehreren der Ansprüche 1 bis 11, **dadurch gekennzeichnet, dass** die Reaktortemperatur, die Katalysator-Kreislauf-Temperatur, die Temperatur des Gemisches aus Frischgas und Kreisgas, die Einsatzmenge an Synthesegas und der Olefinpartialdruck im Reaktor als die bei der Hydroformylierungsreaktion herrschenden Eingangsgrößen für das neuronale Netz verwendet werden.

13. Verfahren gemäß einem oder mehreren der Ansprüche 1 bis 12, **dadurch gekennzeichnet, dass** als Olefin Propylen, Buten-1, Buten-2 oder ein Gemisch enthaltend Buten-1 und Buten-2 verwendet wird.

14. Verfahren nach einem oder mehreren der Ansprüche 1 bis 13, **dadurch gekennzeichnet, dass** die Hydroformylierung in einem homogenen organischen Reaktionsgemisch oder unter Verwendung einer wässrigen Katalysatorlösung nach dem heterogenen Zweiphasenprozeß erfolgt.
